# EUROPEAN PATENT SPECIFICATION

(11) **EP 4 272 015 B1**
(45) Date of publication and mention of the grant of the patent: **19.08.2026**
(21) Application number: 22919141.6
(22) Date of filing: 06.01.2022
(51) Int. Cl.: G01S 7/52, G01S 15/89, A61B 8/00, A61B 8/08, G10K 11/34

(54) **FULL-ARRAY DIGITAL 3D ULTRASOUND IMAGING SYSTEM INTEGRATED WITH A MATRIX ARRAY TRANSDUCER**
MIT EINEM MATRIXARRAY-WANDLER INTEGRIERTES DIGITALES 3D-ULTRASCHALLBILDGEBUNGSSYSTEM MIT VOLLARRAY
SYSTÈME D'IMAGERIE ULTRASONORE 3D NUMÉRIQUE À RÉSEAU COMPLET INTÉGRÉ À UN TRANSDUCTEUR À RÉSEAU MATRICIEL

(43) Date of publication of application: 08.11.2023
(73) Proprietor: Exo Imaging, Inc., Santa Clara, CA 95054 (US)
(72) Inventor: USTUNER, Kutay, Redwood City, California 94065 (US); STEWARD, Chad, Redwood City, California 94065 (US); DEA, David, Redwood City, California 94065 (US); STRODE, Jonathan, Redwood City, California 94065 (US); HAQUE, Yusuf, Redwood City, California 94065 (US); WU, Bicheng William, Redwood City, California 94065 (US); BRADLEY, Charles, Redwood City, California 94065 (US); CAI, Anming, Redwood City, California 94065 (US)
(74) Representative: Viering, Jentschura & Partner mbB Patent- und Rechtsanwälte
(86) International application number: PCT/US2022/011417
(87) International publication number: WO 2023/132829

(56) References cited:
- EP-B1- 0 881 492
- WO-A1-2021/126992
- US-A1- 2007 242 567
- US-A1- 2008 262 357
- US-A1- 2011 225 222
- US-A1- 2014 128 740
- US-A1- 2019 212 424
- US-A1- 2021 267 574
- US-B1- 8 019 016

## Description

### BACKGROUND

The present disclosure relates to systems, devices, and methods for ultrasound imaging, particularly three-dimensional (3D) imaging.

Wide field of view 3D imaging with large steering angles generally requires two- dimensional (2D) (matrix) array transducers with high element density both in azimuth and elevation. High resolution and high sensitivity, on the other hand, generally require wide apertures. Therefore, a good 3D transducer generally requires a very high transducer element count on the order of thousands to tens of thousands of (transducer) elements. High element count creates a major implementation challenge for imaging systems, particularly for receive beam formation, forcing to keep the element count low, and/or limiting the receive beam formation to a multistep beam formation where only the first step, the microbeamformer, is in close proximity to the array or is integrated with it and the second step, the macrobeamformer, is on a remote processor. The microbeamformer generally performs the intra- sub array beamformation and is typically a single-beam analog beamformer often without dynamic focusing capability. The macrobeamformer performs the inter-subarray beamformation and is typically a digital beamformer with dynamic focusing and multibeam (parallel beam) capabilities. The split processing can create issues of connectivity via flex/cables and limits the signal and control data bandwidth.

The following patent references may be relevant: US20210183832A1, US20210028792A1, US20200405271A1, US20200405267A1, US20200405266A1, US20200315586A1, US20190361102A1, US20190299251A1, US20190261954A1, US20190261955A1, US10755692B2, US20180366102A1, US10857567B2, US20180361431A1, US20190196012A1, US20190212424 Al, US11154276B2, US20190133556A1, US10641879B2, US10405829B2, US20160151045A1, US20190388059A1, US20150297193 Al, US20170135676A1, US9592032B2, US20160202349A1, US20160242739A1, US20170296144A1, US20170296145A1, US9521991B2, US20140243676A1, US9439625B2, US20120143059A1, US8545406B2, US20100249596A1, US8416643B2, US8926514B2, US20090326375A1, US8834369B2, US20090240152A1, US8137280B2, US20070016023A1, US20090007414A1, US20050068221A1, US6937176B2, US5928152A, US5675554A, US5685308A, US5555534A, US20010020130A1, US5970025A, EP0881492B1 US2021267574A1, and WO2021 126992 A1

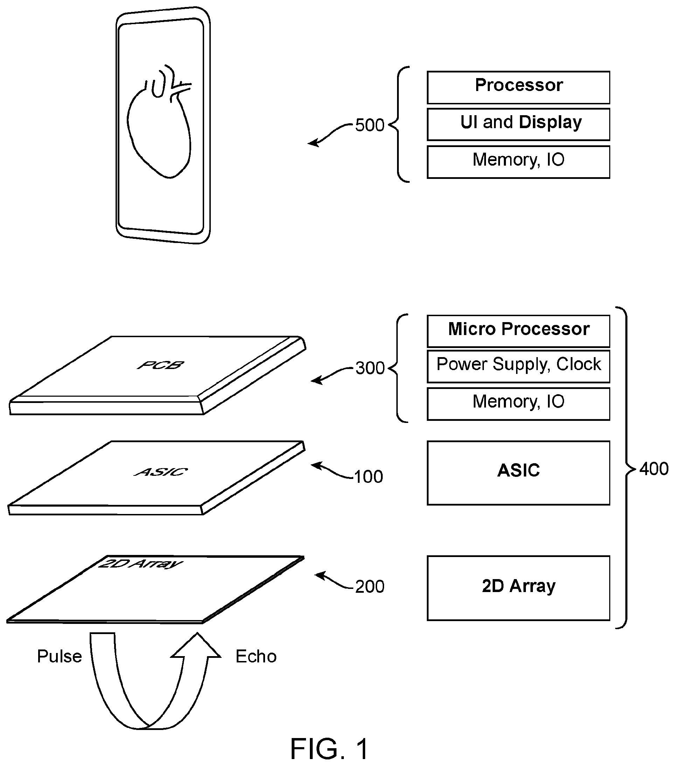

Of these references, EP0881492B1 discloses a hand-held ultrasound imaging system having inter alia a transmit/receive ASIC 20, 20A and a front end ASIC 30, the latter including A/D converters 310, a dynamic focus controller 314, a dynamic weight controller 318, and a summer 320 to provide dynamic focusing of received echo signals. TGC stages 422, 424, 426 are integrated in the transmit/receive ASIC 20A, see figures 1, 5, and 6.

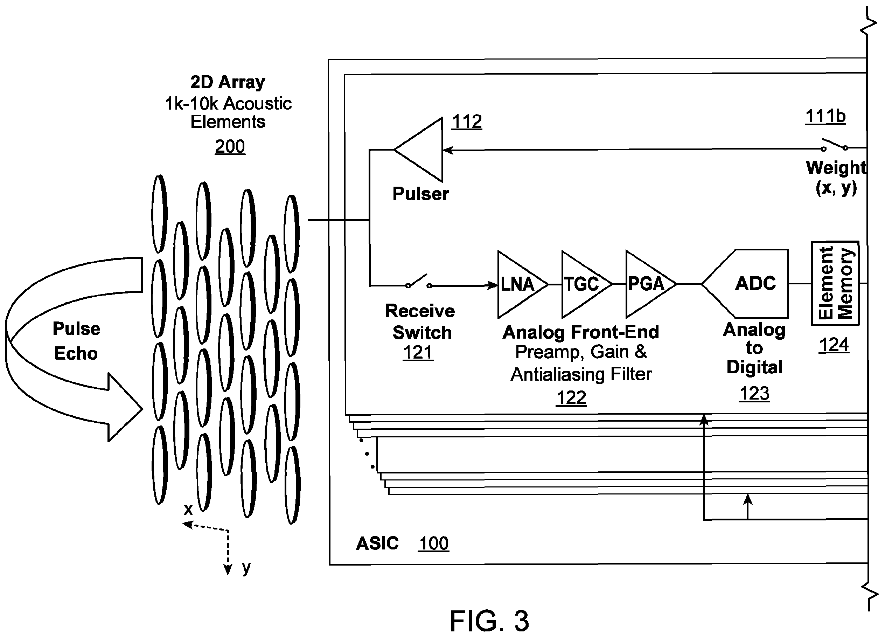

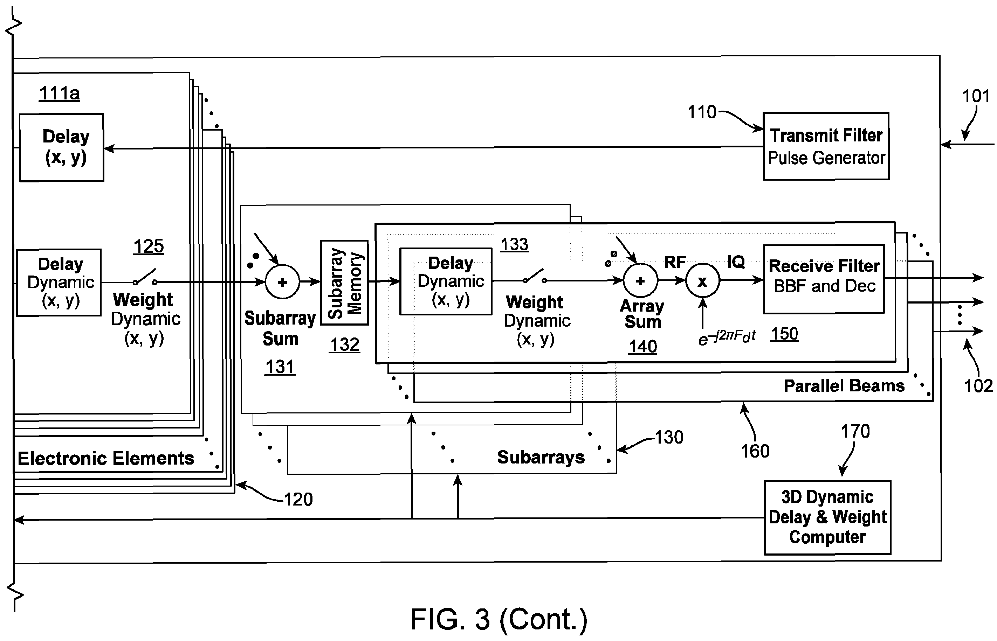

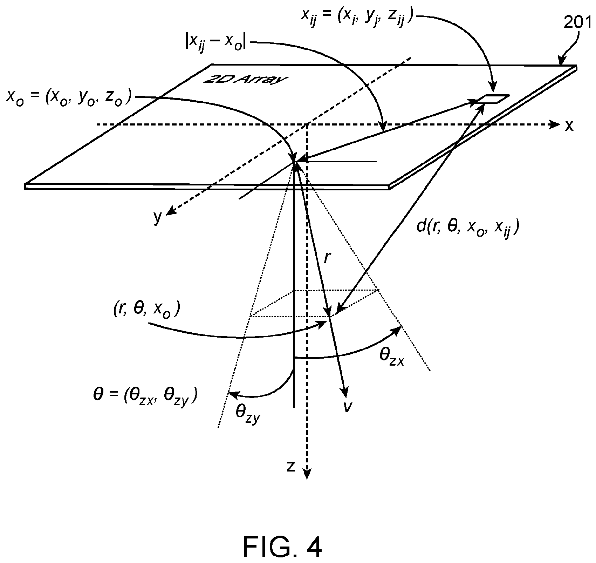

US2021267574A1 discloses a flexible ultrasound phased array including a piezoelectric transducer array and four corresponding slave ICs 325 coupled to a master IC 315. Each slave IC 325 comprises LNA 430, TGC 435, ADC 445, delay and sum units 450, 455, see figures 3 and 4 with paragraphs [0039] to [0041]. The slave ICs obtain phasing and apodization from the master IC so that received signals can be delayed and summed locally at each slave IC and the master IC only needs to sum the result from each slave IC, see paragraph [0040].

WO2021126992A1 discloses ultrasound-on-chip devices comprising two (figure 33: 3302, 3306) or three (figure 34: 3402, 3404, 3406) stacked ASICs, one (3302) of the two ASICs or two (3402, 3404) of the three ASICs comprising cMUT or pMUT transducers, transmission circuitry and analog reception circuitry including analog beamforming circuitry, and ADCs, the remaining ASIC (3306, 3406) having digital receive circuitry including digital beamforming circuitry, see paragraphs [0083], [0086], [0090], and [00107].

### SUMMARY

The invention is set out in the appended set of claims. The present disclosure relates to systems, devices, and methods for ultrasound imaging, in particular to 3D imaging with massive number of transducer elements.

The present disclosure relates to methods for a full-array digital 3D transmit and receive beamformer that can be integrated on an application specific integrated circuit (ASIC) which in turn can be integrated on a high element count 2D array transducer. This may reduce cost, size, weight and power of an ultrasound imaging system.

An aspect of the present disclosure is that the analog signal of every element of a 2D array is digitized by a N-bit ADC at a sampling rate of Fs after preamplification. In some embodiments, a single bit ADC, e.g., a simple comparator, with a sampling rate that is 16 times the imaging center frequency is used (Fs = 16F0 ). Using single bit ADCs can simplify the beamforming architecture significantly, reducing cost and power. Sampling at 16F0 can allow high quality dynamic receive beam formation with T0 /16 delay quantization steps without the need for up-sampling. As an example, a 4,096-element array with a per-element dithered 1 -bit ADC operating at 16 times the imaging frequency would have 56dB digital dynamic range for an imaging BW that is equal to the imaging frequency.

Another aspect of the present disclosure is that the on-ASIC dynamic receive beamformer may generate multiple beams in response to each transmit event which can be essential for high volume rate imaging.

Another aspect of the present disclosure relates to an on-ASIC delay and weight engine which may generate the delay and weight for each element and for each depth for dynamic receive beamformation. This can reduce the amount of control data needed by the ASIC significantly since the ASIC can generate any arbitrary beam with only a few input parameters, namely the beam origin, beam angle and f-number. This can simplify the off-ASIC circuitry significantly and can reduce the interconnect bus width and bandwidth. In a preferred embodiment, the same delay and weight engine is used also to create the delay and weight profiles for transmit beamformation

Another aspect of the present disclosure relates to methods for ultrasound imaging and beam forming with a matrix array of transducer elements. In a step (a), receive signals of each transducer array element may be amplified. In a step (b), the amplified receive signal of each transducer array element may be digitized. In a step (c), a delay and weight may be applied on the amplified and digitized receive signals. In a step (d), the amplified, digitized, delayed, and weighted receive signals may be summed across all transducer elements of the matrix array to form a dynamically focused receive beam

Another aspect of the present disclosure relates to systems for ultrasound imaging. An exemplary system may comprise a matrix array of transducer elements and circuitry with the matrix array. The circuitry may be configured to: (a) amplify receive signals of each transducer array element, (b) digitize the amplified receive signal of each transducer array element, (c) apply a delay and weight on the amplified and digitized receive signals, and (d) sum across all transducer elements of the matrix array the amplified, digitized, delayed and weighted receive signals to from a dynamically focused receive beam.

Another aspect of the present disclosure relates to methods and systems for ultrasound beamforming with a matrix array of transducer elements.

In an exemplary method, a delay may be applied on a receive signal from the matrix array by performing at least one CORDIC (COordinate Rotation DIgital Computer) operation. The at least one CORDIC operation may comprise two cascading CORDIC operations. The two cascading CORDIC operations may comprise a first CORDIC operation and a second CORDIC operation, and an output of the first CORDIC operation may be an input to the second CORDIC operation. The at least one CORDIC operation may be performed by an application specific integrated circuit (ASIC) operatively coupled to the matrix array. Delays for each transducer element of the matrix array may be determined for a subset of depths with the at least one CORDIC operation. Delays for in-between depth grid points may be interpolated. Delays for in-between elements may be interpolated. Delays for in-between beams may be interpolated.

In an exemplary system, the system may comprise a matrix array of transducer elements and circuitry coupled with the matrix array and configured to perform the aforementioned exemplary method.

### BRIEF DESCRIPTION OF THE DRAWINGS

A better understanding of the features and advantages of the present disclosure will be obtained by reference to the following detailed description that sets forth illustrative embodiments and the accompanying drawings.
**Fig. 1** shows an exemplary schematic diagram of an ultrasonic system using a transducer assembly comprised of a 2D array of transducers and an ASIC mounted on a PCB with additional circuitry, and a remote processor with a user interface and display.
**Fig. 2** shows a schematic of a digital 3D single-stage full-array beamformer with an ASIC.
**Fig. 3** shows a schematic of a digital 3D two-stage full-array beamformer with an ASIC.
**Fig. 4** shows a graph of the geometry of ultrasound beams generated by an ultrasound transducer array.
**Fig. 5** shows a flow chart of a 3D dynamic delay and weight computer.

### DETAILED DESCRIPTION

Unless otherwise defined, all technical terms used herein have the same meaning as commonly understood by one of ordinary skill in the art to which the present subject matter belongs.

### Ultrasound Imaging System

**Fig. 1** shows an exemplary embodiment of the ultrasonic imaging system disclosed herein. The imaging system may include an ASIC **(100)** preferably integrated with a transducer **200.** The transducer may be a one-dimensional or a two-dimensional array of pMUT (piezoelectric micromachined ultrasound transducer), cMUT (capacitive micromachined ultrasound transducer), or bulk PZT elements. The ASIC and transducer array are typically mounted on a PCB (or PCBs) **(300).** The PCB may have additional circuitry such as a microprocessor, power supply (battery, regulators), clock, memory and an input output device.

The ASIC, transducer array, and the PCB form a transducer assembly **(400).** The area of the transducer assembly may match the area of the transducer array to keep the footprint small. The transducer assembly can be packaged in a patch, or in a wearable or holdable housing.

The transducer assembly, via an input output device, may communicate with a remote processor **(500)** that may include a user interface, display and memory. The processor may be a mobile device such as a smart phone, smart watch, pad or a laptop, or it can be a desktop computer. It may perform image processing, perform plane and volume rendering, and connect to a network and database such as electronic health records. The communication between the transducer assembly and the remote processor may be wired or wireless, using standard communication protocols.

The microprocessor on the transducer assembly may initialize the ASIC with a small set of parameters such as the imaging frequency and the transmit and receive f-numbers and then may provide the transmit and receive beam parameters (beam origin, angle, focus depth) for each pulse-echo (transmit-receive) event in the scan sequence. An on-ASIC delay and weight computer may compute the transmit and receive beamforming parameters (delay and weight) for each beam defined by the transmit and receive beam parameters. The ASIC may send out a steered and focused transmit pulse, may receive the echo from tissue at each transducer element, and may form receive beams using ASIC-computed delay and weight. The output of the ASIC are typically the fully formed beams using the full aperture.

The sections below describe the transducer assembly, the transmitter and receiver, the geometry used for the derivation of a 3D delay equation, and a method and device for the delay and weight computation using the 3D delay equation.

### Transducer Assembly

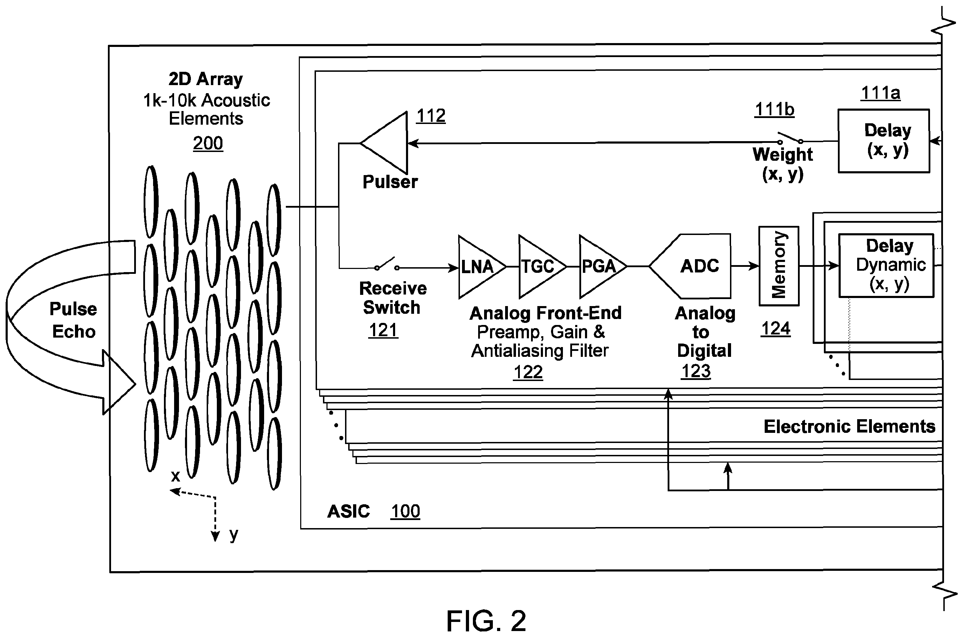

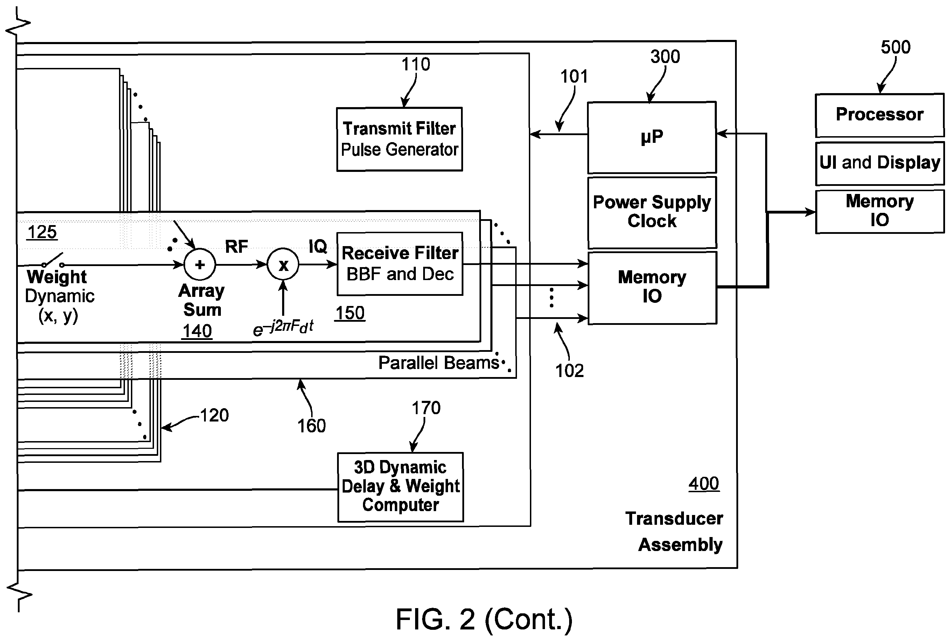

**Fig. 2** shows the details of the transducer assembly **(400),** and the ASIC **(100)** within the transducer assembly. The ASIC receives inputs **(101)** from the microprocessor on the PCB **(300).** The inputs may include initialization parameters such as transmit center frequency and bandwidth, transmit and receive f-number, and receive center frequency and bandwidth. The ASIC may also receive transmit and receive beam parameters and a trigger for each pulse-echo event. The transmitter may create the transmit pulse **(110),** apply an element coordinate dependent delay **(111a)** and weight **(111b)** to the pulse , and drive the pulser **(112)** of each acoustic element with the delayed and weighted pulse based on the transmit pulse and transmit beam parameters.

The receive path for each acoustic element can contain a transmit/receive switch **(121),** an analog front end **(122)** for low noise preamplification, time gain compensation and antialiasing, an ADC **(123),** an element memory **(124),** and a beamformer **(125)** that may apply time varying (dynamic) delay and weight on the stored element data. The transmit beamformer (delay and weight), pulser, receive switch, analog front-end, ADC, memory and receive beamformer (delay and weight) circuitry may form an electronic element **(120).** There may be an electronic element per acoustic element.

The outputs of the electronic elements can be summed across the whole array **(140)** to complete the full array beamformation. The beams thus formed may then be filtered by a receive filter **(150)** for data compression which may comprise demodulation to baseband by a complex time varying multiplier followed by a lowpass baseband filter (BBF). The delay, weight, array sum and receive filter circuitry may be duplicated to form multiple beams with distinct delay and/or weight parameters in parallel **(160)** using the same element data stored in the memory. The delay and weight for the transmit and receive beamformation (for all parallel beams) may be created by an on-ASIC 3D Dynamic Delay and Weight Computer **(170).** The outputs of the ASIC **(102)** may be the complex (in phase and quadrature phase) samples of parallel beams. The transducer assembly stores the output beams and sends them to a remote processor **(500)** for further processing, rendering, and display.

The receive beamformation of **Fig. 2** can also be implemented in multiple stages. **Fig. 3** shows a two-stage version. The multi-stage implementation allows flexibility to reduce the size of both the element memory and the parallel beam circuitry. Instead of summing the outputs of all electronic elements, the outputs of subsets of electronic elements (subarrays) **(130)** may be summed **(131)** and stored in a second set of subarray memory **(132).** Note that the first stage beamformation within each subarray may also be known as micro beamformation. A second stage applies delay and weight **(133)** on the subarray beamformer outputs, and the array sum **(140)** may complete the full array beamformation. Only the second stage circuitry (macro beamformer) may be duplicated for parallel beam operation. The subarray size can be *Sₓ* × *S_{y}* elements where *Sₓ* and *S_{y}* can be 2, 3, 4, 5, etc.,... electronic elements.

### Transmitter

A single K-bit deep, L-bit long shift register with a programmable clock can serve as an arbitrarily programmable pulse generator **(110).**

The depth of the shift register K may be determined by the number of pulser states. In general, a K-bit deep shift register can support pulsers up to 2^{K} states. So K would be 1 for 2-state (unipolar) pulsers, 2 for a 3-state (bipolar) and 4-state pulsers, and so on.

The length L of the shift register may be determined by the maximum pulse length spec and the transmitter clock frequency. In a preferred embodiment, the shift register length L is set to 256 bits. This would support up to 16-cycle long pulses at a transmit clock cycle that is 16 times the transmit center frequency. Longer than 16-cycle pulses can still be supported by lowering the transmitter clock frequency (trading off delay quantization step).

The simplest type of pulses may be unipolar pulses where the active node of the transducer element is altered between a ground and a positive (or negative) voltage rail by two complementary switches. These switches can be controlled by a single one-bit stream, a set of 1s for +V segment followed by a set of 0s for GND, where this pattern of 1s and 0s is repeated as many cycles as needed. Each bit may represent duration of a transmitter clock cycle. So if transmitter clock cycle is 16 *F*₀ the bit stream of a two-cycle pulse at *F*₀ would be 11111111000000001111111100000000. The durations of the individual +V and GND segments can be fixed or independently programmable, e.g., for linear (or nonlinear) frequency modulation, or some other coded excitation. Such a bit pattern can be generated in advance and loaded to the pulse generator shift register in the ASIC during initialization and streamed out upon receiving the impulse indicating the start of transmit. In some embodiment, the start and/or end of the pulses can be marked by a very short code such as 010, e.g., 11111111000000001111111100000000010, to trigger other transmit and/or receive circuitry on or off. Utilization of such an embedded code may require a decoder (Matched Filter) of the same length. In some embodiments, the transmit/receive switch of each element may be turned on to the receive mode on as soon as the element's own pulse transmission is complete without waiting for all elements to finish pulse transmission. This can help clean up some of the near field artifacts by temporally dispersing the leaked transmit and receive enable/disable signals and eliminate dead zones due to missed receive samples.

Next in complexity are the 3-state bipolar pulses where the active node of the transducer element is altered between a positive, ground, and a negative voltage rail by three complementary switches. This type of pulses can be implemented using a 2-bit deep pulse stream where for example 00 indicates ground, 10 indicates +V, and 01 indicates -V. The 11 state can be utilized to mark the start and/or end of the pulse.

A special case of the 3-state bipolar pulses is where the transducer is grounded only before the pulse starts and after the pulse ends and switched between the +V and -V states during the pulse. This type of pulse may offer the best second harmonic suppression compared to all 2-state pulses and those 3-state pulses with ground segments within the pulse. It may also be the simplest (lowest cost) architecture in terms of power supplies. This special case of bipolar pulses can be implemented using a single bit stream above where 1 is mapped say to +V and 0 to the -V. The embedded snippet of code described above can be used to indicate the start of the ground state at the end of the pulse. Upon reception of this code the transducer element is grounded until the start of the next pulse which is indicated by a stream of 1's. The pulse inversion capability can be added with an additional programmable bit that is common to all elements that inverts the mapping of the 1 and 0 values to -V and +V at the pulser.

A pulse common to all elements can be generated upon an impulse marking the onset of a pulse-echo event which is typically repeated at regular Pulse Repetition Intervals (PRI). Then, the pulse may be delayed **(111a)** by an element specific delay for every element of the array. The delayed pulse can then be weighted by an element specific weight for apodization. Here, a simple binary on/off weight is shown. In a preferred embodiment both the delay and weight of transmit beamformer are generated by the on-ASIC delay and weight computer **(170)** before the transmit event starts.

The output of the apodization can drive the transmit pulsers **(112)** after a digital to analog conversion.

In some embodiments, the pulse generator and delay operation share the same transmitter clock for architectural simplicity. Further, for efficiency purposes, the transmitter clock frequency *Fₛ* can be varied as a function of the transmit center frequency *F*₀ and may be set equal to 16 *F*₀ to achieve a desired delay quantization step of *T*₀/16, where *T*₀ = 1/*F*₀.

In some embodiments, the order of the pulse generator, delay, and binary weight can be changed. For example, the binary weight can be moved before the delay operation or the delay operation can be moved before the pulse generator, etc., for various architectural tradeoffs.

### Receiver

A typical receiver applies a dynamically varying gain, delay, and weight (apodization) on the echo from individual elements *sᵢⱼ*(*t*), where (i, j) are the column and row indices of the elements of a matrix array. Then, the beamformer may sum the amplified, delayed, and weighted element signals to generate a beam *b*(*r, **θ**, **x_{O}***) where ***x_{O}*** is the (*x_{O}, y_{O}, z_{O}*) coordinates of the beam origin (*z_{O}* is zero for planar arrays), *r* is depth and ***θ*** is the beam angle in z-x and z-y planes. For a digital beamformer, the analog signal may be converted to digital by an ADC after the LPF before the delay stage. $b \left(r, θ, {x}_{O}\right) = {\sum }_{i} {\sum }_{j} G \left(t-τ\left(r, θ, {x}_{O}, {x}_{ij}\right)\right) a \left(r, θ, {x}_{O}, {x}_{ij}\right) {s}_{ij} \left(t-τ\left(r, θ, {x}_{O}, {x}_{ij}\right)\right)$

The gain *G*(*t*) may have multiple programmable components including a static Low Noise Amplifier gain G_{LNA}, and a dynamic time-varying gain *G_{TGC}*(*t*) (also referred to as Time Gain Compensation) to compensate for tissue attenuation. The last gain stage may be an optional Programmable Gain Amplifier.

A low-pass filter (LPF) with a preferably programmable cut-off frequency provides antialiasing and improves SNR. The multiple poles of the LPF can be distributed among the various gain stages.

The dynamic delay *τ*(*r, **θ***, ***x_{O}**, **xᵢⱼ***) may vary with time to track the depth the echo is coming from as the transmit beam propagates deeper into the tissue. The input of the delay stage is a function of time, while the output of it is a function of depth (range). Depth is warped time due to the time varying delay.

The dynamic apodization or weight *a*(*r, **θ**, **x_{O}**, **xᵢⱼ***) may grow the active aperture size with depth to preserve resolution and tapers the contribution of edge elements, i.e., apodizes to reduce the beam sidelobes. For matrix arrays, the active aperture shape may also have an apodization effect. In some embodiments, the apodization weight is depth-dependent but it is binary, 0 for off and 1 for on, eliminating the need for a multiplication per element and per depth. By turning on elements around the beam origin within an ever-growing circle or ellipsoid a half-circle like apodization is achieved. The growth rate of the circle and ellipse may be controlled by a programmable f-number. Since *G_{TGC}* is applied before the delay operation, the gain may be dispersed in time as a function of the element-dependent delay. This can create an additional apodization effect for depths the gain changes rapidly.

The dynamic delay and weight computations may be performed by a computer given the beam parameters ***θ*** and ***x_{O}**,* element coordinates ***xᵢⱼ***, ADC sampling rate *Fₛ*, speed of sound *c*₀ and f-number. In many prior art systems, these computations are done fully or partially on remote processors.

The element sum stage may sum the time-aligned (therefore coherent) and weighted element signal.

Multiple beams with independent origins and angles can be generated in parallel using a duplicated set of delay, weight, and element sum stages . Alternatively, if the element data is stored for the full depth of interest, multiple beams can be formed serially using a single beamformer circuitry using the time in between transmit events, trading off frame rate.

### Array and Beam Geometry

**Fig. 4** depicts a *Nₓ × N_{y}* - element 2D array **(201)** on an x-y plane (or on a nonplanar curved x-y-z surface, which is not shown in Fig. **4****)** centered at the (0,0,0) of the Cartesian coordinates. ***xᵢⱼ*** is the x, y, and z coordinates (*xᵢ, yⱼ, zᵢⱼ*) of its (i, j)^{th} element. The elements of the 2D array can be on a square or rectangular grid, rotated square, rhombus (parallelogram), hexagonal, annular, or an arbitrary grid. The physical aperture can be a square, rectangle, circle or ellipse, or an arbitrary shape.

A beam can be defined in 3D by three parameters: a focusing depth r for the static transmit focus, or a set of focusing depths for the dynamic receive focus, a (nominal) beam origin ***x_{O}*** which is a vector of its x, y, and z coordinates ***x_{O}*** = (*x_{O}, y_{O}, z_{O}*) and the angle ***θ*** which is also a vector of its z-x plane and z-y plane angles ***θ** =* (*θ_{zx}, θ_{zy}*). Note that we here use bold letters to represent vectors such as ***x_{O}*** and ***θ***. The coordinates of a sample along a receive beam (***θ***, ***x_{O}***) at depth (or range) r is (*r, **θ**, **x_{O}***). The convention for ***θ*** is such that *θ_{zx}* and *θ_{zy}* are positive from the +z axis to the +x and +y axes, respectively. The beam origin ***x_{O}*** is also the depth zero (*r =* 0). It is also the nominal center of the active aperture for beam (***θ***, ***x_{O}***) excluding the truncation by the physical aperture. All samples of the receive beam lie on the line the projections of which are at angles *θ_{zx}* and *θ_{zy}* on the z-x and z-y planes respectively.

2D imaging in the azimuth (i.e., x-z) plane is a special case where *θ_{zy}* and *y_{O}* are zero for all beams. 2D imaging in the orthogonal elevation (y-z) plane corresponds to the case where *θ_{zx}* and *x_{O}* are zero. A special case of 2D imaging is where the array is a 1-D array, e.g., *N_{y} =* 1.

The geometry defined here can support independent combinations of scan geometries for the azimuth and elevation. For example, to define a Sector geometry both in azimuth and elevation, *x_{O}* and *y_{O}* would both be set to 0 for all beams. For a Linear scan, say in elevation, *θ_{zy}* would be set to zero for all beams while varying *y_{O}* from the first row to the last. For a Vector format, such as in elevation, *θ_{zy}* would be varied from a negative angle to a positive one while varying *y_{O}* from the first row to the last.

The geometry here can apply to multi-stage beamformation as well where a first stage subarray beamformer (micro beamformer) performs beamformation on groups of *Sₓ* × *S_{y}* elements, and a second stage *Mₓ × M_{y}* beamformer (macro beamformer) completes the beamformation on the outputs of the subarray beamformers where *Nₓ* = *Sₓ Mₓ* and *N_{y} = S_{y} M_{y}.*

Note that there are alternative coordinate systems to define the beams in 3D such as the Spherical Coordinates. The relationships between the angles (*θ*, *φ*) of the Spherical Coordinates centered at beam origin ***x_{O}*** and the beam angles (*θ_{zx}, θ_{zy}*) of the framework adapted here are: ${θ}_{zx} = {tan}^{- 1} \left(tan θ sin φ\right)$ ${θ}_{zy} = {tan}^{- 1} \left(tanθ cosφ\right)$ $θ = {tan}^{- 1} \left({\left({\left({tanθ}_{zx}\right)}^{2}+{\left({tanθ}_{zy}\right)}^{2}\right)}^{1 / 2}\right)$ $φ = {tan}^{- 1} \left(\frac{{tanθ}_{zy}}{{tanθ}_{zx}}\right)$

The analysis and derivations here can apply to any alternative beam definition with minor modifications.

### 3D delay equation

The distance *d*(*r*, ***θ***, ***x_{O}***, ***xᵢⱼ***) for depth r along the beam (***θ***, ***x_{O}***) for a particular element (i, j) can now be derived.

The Cartesian coordinates (*b_{y}, b_{y}, b_{z}*) of the beam sample (*r, **θ**, **x**_{O}*) are $\left({b}_{x}, {b}_{y}, {b}_{z}\right) = r \left({v}_{x}, {v}_{y}, {v}_{z}\right) + \left({x}_{O}, {y}_{O}, {z}_{O}\right)$
where, the unit vector ***v*** = (*vₓ, v_{y}, v_{z}*) along the beam is $v = \left({v}_{x}, {v}_{y}, {v}_{z}\right) = \frac{\left({tanθ}_{zx}, {tanθ}_{zy},1\right)}{\sqrt{{tan}^{2} {θ}_{zx} + {tan}^{2} {θ}_{zy} + 1}}$
and the x, y, z coordinates of the beam are ${b}_{x} = r {v}_{x} + {x}_{O} , {b}_{y} = r {v}_{y} + {y}_{O} , {b}_{z} = r {v}_{z}$

Then the distance between ***xᵢⱼ*** = (*xᵢ, yⱼ*) and (*r, **θ**, **x_{O}***) = (*bₓ, b_{y}, b_{z}*) is given by $d \left(r,θ,{x}_{O},{x}_{ij}\right) = \sqrt{{\left({x}_{i}-{b}_{x}\right)}^{2} + {\left({y}_{j}-{b}_{y}\right)}^{2} + {\left({z}_{ij}-{b}_{z}\right)}^{2}}$

The square root of the summation of the squares of three terms can be written as the square root of the summation of the squares of two terms as follows. $d \left(r,θ,{x}_{O},{x}_{ij}\right) = \sqrt{{\left({x}_{i}-{b}_{x}\right)}^{2} + {\left(\sqrt{{\left({y}_{j}-{b}_{y}\right)}^{2} + {\left({z}_{ij}-{b}_{z}\right)}^{2}}\right)}^{2}}$

Delay *τ*(*r, **θ**, **x_{O}**, **xᵢⱼ***) in µs is the distance *d*(*r, **θ**, **x_{O}**, **xᵢⱼ***) in mm divided by the roundtrip (two-way) speed of sound *c*₀ in mm/µs. $τ \left(r, θ, {x}_{O}, {x}_{ij}\right) = d \left(r, θ, {x}_{O}, {x}_{ij}\right) / \left({c}_{0}/2\right)$ or in units of the number of samples at the ADC sampling rate of *Fₛ* in MHz $τ \left(r, θ, {x}_{O}, {x}_{ij}\right) = {F}_{s} d \left(r, θ, {x}_{O}, {x}_{ij}\right) / \left({c}_{0}/2\right)$

### 3D Dynamic Delay and Weight Computer

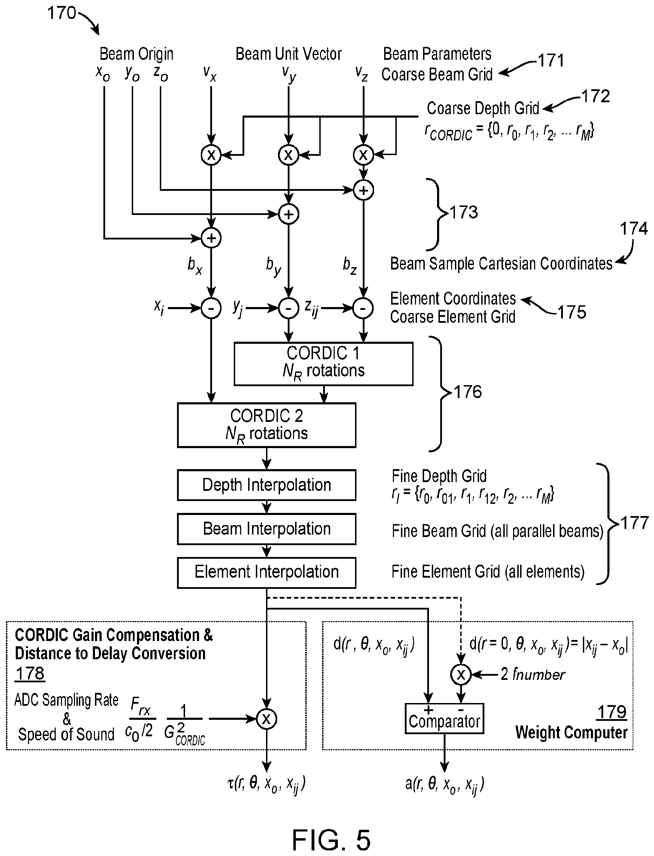

The delay formulation above lends itself to an efficient implementation using CORDIC (COordinate Rotation DIgital Computer) which is an efficient method to compute the square root of the squares of two numbers. **Fig. 5** shows the block diagram and steps of a dynamic 3D delay and weight computer **(170)** using two cascaded CORDIC operations **(176).**

The inputs to the delay and weight computer may include the beams' origin, unit vector and focus depth(s), the coordinates of the elements, ADC sampling rate, the speed of sound, and f-number.

The beam unit vector Cartesian coordinates **(171)** may be multiplied with depth **(172)** and added to the beam origin coordinates **(173)** to create the beam sample Cartesian coordinates for a particular depth r **(174).** The x, y, and z coordinates of the elements may be subtracted from the respective x, y, and z coordinates of the beam sample **(175)** to create the inputs to the CORDIC operations. The output of the first CORDIC and the x component of the beam sample may form the inputs of the second CORDIC. The output of the second CORDIC may provide the distance between the element (i, j) and the beam sample (*r, **θ**, **x_{O}***) scaled by the gain of the two CORDIC stages (CORDIC is not a unit-gain operation). In a preferred implementation, the CORDIC gain compensation may be performed by the distance to delay conversion multiplier that is at the output of the delay computer **(178).**

In some embodiments, the cascaded CORDICs do 8 angle rotations each. This number of rotations may be sufficient to bring the maximum distance error within *T*₀/16, where *T*₀ is the period at the imaging center frequency *F*₀. Each angle rotation may take 2 bit shifts and 2 additions. For eight angle rotations, each CORDIC stage has a gain that is equal to ~1.65, and the two CORDIC stages together have a total gain of ~2.71.

Note that the CORDIC based high precision distance (delay) computations may be needed only for a sparse set of depths, elements, and beams. A linear interpolation between the CORDIC computed distance values **(177)** may be sufficient to keep the delay error within specifications. In some embodiments, the coarse range grid is spaced $4 {λ}_{0} {f}_{number}^{2}$, where *λ*₀ is the wavelength at imaging center frequency *F*₀. A linear distance interpolator may provide the distance values mid-point between the coarse range grid points. In some embodiments, CORDIC based delay computations are performed for a subset of beams, e.g., edge beams of a multibeam group, and a linear distance interpolator may provide the distance values for the in-between beams. In some embodiments, the coarse element grid is spaced 4 elements apart both in azimuth and elevation. Again, a linear distance interpolator may interpolate the distance values for the in-between elements. Since linear interpolations for powers of 2 up-sampling require only additions and bit shifts, they can be very efficient.

The last stage of the delay engine **(178)** may compensate for the non-unity gain of the CORDIC stages and converts the distance *d*(*r, **θ**, **x_{O}**, **xᵢⱼ***) which is in a unit of mm to delay *τ*(*r, **θ**, **x_{O}**, **xᵢⱼ***) in a unit of the ADC sample rate using the ADC sampling rate and speed of sound as the inputs. Having the distance to delay conversion at the very output may allow an easy way of optimizing the bulk speed of sound as a function of clinical application and the ADC sample rate as a function of the imaging center frequency.

The order of linear operations are interchangeable. For example, the distance to delay conversion can be done at any point in the delay computer signal path, or the interpolations can be reordered depending on implementation specific concerns.

In some embodiments, the weights are binary, i.e., an element is either on or off at any particular time/depth. The delay computer may provide the inputs to the weight computer. Note that the distance between any element and the beam origin can be computed by the delay computer by setting r to zero, l***xᵢⱼ** - **x_{O}***| = d(***r*** = **0**, ***θ**, x_{O}*, *xᵢⱼ*). This distance scaled by a scalar that is a function of the f-number (aperture growth rate) can be compared to the distance output of the delay computer during receive event to turn each element on at the right time (depth) **(179).** With this method the aperture can be grown as a circle around the beam origin. Alternatively, both the growth rate and the aperture limits can be programmed for x and y independently, say for rectangular or ellipsoidal aperture growth.

While preferred embodiments have been shown and described herein, it will be obvious to those skilled in the art that such embodiments are provided by way of example only. Numerous variations, changes, and substitutions will now occur to those skilled in the art It should be understood that various alternatives to the embodiments described herein may be employed in practice. Numerous different combinations of embodiments described herein are possible. In addition, all features discussed in connection with any one embodiment herein can be readily adapted for use in other embodiments herein.

## Claims

1. A method for ultrasound imaging and beam forming with a matrix array of transducer elements (200), the method comprising:
a) amplifying, by an application specific integrated circuit, ASIC, (100) integrated with the matrix array of transducer elements (200), receive signals of each transducer array element;
b) digitizing, by the ASIC, the amplified receive signal of each transducer array element;
c) applying, by the ASIC, a delay and weight on the amplified and digitized receive signals; and
d) summing, by the ASIC, across all transducer elements of the matrix array the amplified, digitized, delayed, and weighted receive signals to form a dynamically focused receive beam.

2. The method of Claim 1, wherein the ASIC also forms transmit beams.

3. The method of any one of Claims 1-2, where a single receive beam is formed per transmit event.

4. The method of any one of Claims 1-3, where two or more receive beams are formed per transmit event.

5. The method of any one of Claims 1-4, wherein the matrix array is comprised of one or more cMUT transducer elements.

6. The method of any one of Claims 1-5, wherein the matrix array is comprised of one or more pMUT transducer elements.

7. The method of any one of Claims 1-6, wherein amplifying the receive signals applies a depth varying amplification gain to the receive signals.

8. The method of any one of Claims 1-7, wherein a N-bit ADC (123) digitizes the amplified receive signal at a sampling rate Fs.

9. The method of any of Claims 1-8, wherein at least one on-ASIC weight computer computes the weights for each element and for each range sample based on depth, f-number and the distance between the element and a beam origin.

10. A system for ultrasound imaging, the system comprising:
i. a matrix array of transducer elements (200); and
ii. circuitry comprising an application specific integrated circuit, ASIC, (100) integrated with the matrix array of transducer elements (200) and configured to:
a) amplify, by the ASIC, receive signals of each transducer array element;
b) digitize, by the ASIC, the amplified receive signal of each transducer array element;
c) apply, by the ASIC, a delay and weight on the amplified and digitized receive signals; and
d) sum, by the ASIC, across all transducer elements of the matrix array the amplified, digitized, delayed and weighted receive signals to from a dynamically focused receive beam.

## Patentansprüche

1. Verfahren zur Ultraschallbildgebung und Strahlformung mit einer Matrixanordnung von Wandlerelementen (200), das Verfahren aufweisend:
a) Verstärken, durch eine anwendungsspezifische integrierte Schaltung (ASIC) (100), die in die Matrixanordnung von Wandlerelementen (200) integriert ist, von Empfangssignalen jedes Wandlerarray-Elements;
b) Digitalisieren, durch die ASIC, des verstärkten Empfangssignals jedes Wandlerarray-Elements;
c) Anwenden, durch die ASIC, einer Verzögerung und einer Gewichtung auf die verstärkten und digitalisierten Empfangssignale; und
d) Summieren, durch die ASIC, der verstärkten, digitalisierten, verzögerten und gewichteten Empfangssignale über alle Wandlerelemente der Matrixanordnung, um einen dynamisch fokussierten Empfangsstrahl zu bilden.

2. Verfahren nach Anspruch 1, wobei die ASIC auch Sende-Strahlen bildet.

3. Verfahren nach einem der Ansprüche 1 bis 2, wobei pro Sendeereignis ein einzelner Empfangsstrahl gebildet wird.

4. Verfahren nach einem der Ansprüche 1 bis 3, wobei pro Sendeereignis zwei oder mehr Empfangsstrahlen gebildet werden.

5. Verfahren nach einem der Ansprüche 1 bis 4, wobei die Matrixanordnung einen oder mehrere cMUT-Wandlerelemente aufweist.

6. Verfahren nach einem der Ansprüche 1 bis 5, wobei die Matrixanordnung einen oder mehrere pMUT-Wandlerelemente aufweist.

7. Verfahren nach einem der Ansprüche 1 bis 6, wobei beim Verstärken der Empfangssignale eine in der Tiefe variierende Verstärkung auf die Empfangssignale angewendet wird.

8. Verfahren nach einem der Ansprüche 1 bis 7, wobei ein N-Bit-ADC (123) das verstärkte Empfangssignal mit einer Abtastrate Fs digitalisiert.

9. Verfahren nach einem der Ansprüche 1 bis 8, wobei mindestens ein On-ASIC-Gewichtsrechner die Gewichte für jedes Element und für jede Entfernungsabtastung auf der Grundlage der Tiefe, der f-Zahl und des Abstands zwischen dem Wandlerelement und einem Strahlursprung berechnet.

10. System zur Ultraschallbildgebung, das System aufweisend:
i. ein Matrixarray von Wandlerelementen (200); und
ii. eine Schaltung, die eine anwendungsspezifische integrierte Schaltung (ASIC) (100) aufweist, die in die Matrixanordnung von Wandlerelementen (200) integriert ist und eingerichtet zum:
a) Verstärken, durch die ASIC, von Empfangssignalen jedes Wandlerarray-Elements;
b) Digitalisieren, durch die ASIC, des verstärkten Empfangssignals jedes Wandlerarray-Elements;
c) Anwenden, durch die ASIC, einer Verzögerung und einer Gewichtung auf die verstärkten und digitalisierten Empfangssignale; und
d) Summieren, durch die ASIC, der verstärkten, digitalisierten, verzögerten und gewichteten Empfangssignale über alle Wandlerelemente der Matrixanordnung hinweg, um einen dynamisch fokussierten Empfangsstrahl zu bilden.

## Revendications

1. Procédé d'imagerie par ultrasons et de formation de faisceau à l'aide d'un réseau matriciel d'éléments transducteurs (200), le procédé comprenant :
a) l'amplification, par un circuit intégré spécifique à une application, ASIC, (100) intégré au réseau matriciel d'éléments transducteurs (200), de signaux de réception de chaque élément de réseau de transducteurs ;
b) la numérisation, par l'ASIC, du signal de réception amplifié de chaque élément de réseau de transducteurs ;
c) l'application, par l'ASIC, d'un retard et d'une pondération aux signaux de réception amplifiés et numérisés ; et
d) la sommation, par l'ASIC, sur l'ensemble des éléments transducteurs du réseau matriciel, des signaux de réception amplifiés, numérisés, retardés et pondérés afin de former un faisceau de réception focalisé de manière dynamique.

2. Procédé selon la revendication 1, dans lequel l'ASIC forme également des faisceaux d'émission.

3. Procédé selon l'une quelconque des revendications 1 à 2, dans lequel un seul faisceau de réception est formé par événement d'émission.

4. Procédé selon l'une quelconque des revendications 1 à 3, dans lequel deux ou plusieurs faisceaux de réception sont formés par événement d'émission.

5. Procédé selon l'une quelconque des revendications 1 à 4, dans lequel le réseau matriciel est constitué d'un ou de plusieurs éléments transducteurs cMUT.

6. Procédé selon l'une quelconque des revendications 1 à 5, dans lequel le réseau matriciel est constitué d'un ou de plusieurs éléments transducteurs pMUT.

7. Procédé selon l'une quelconque des revendications 1 à 6, dans lequel l'amplification des signaux de réception applique aux signaux de réception un gain d'amplification variant en fonction de la profondeur.

8. Procédé selon l'une quelconque des revendications 1 à 7, dans lequel un ADC à N bits (123) numérise le signal de réception amplifié à une fréquence d'échantillonnage Fs.

9. Procédé selon l'une quelconque des revendications 1 à 8, dans lequel au moins un calculateur de pondération sur l'ASIC calcule les pondérations pour chaque élément et pour chaque échantillon de portée en fonction de la profondeur, du nombre f et de la distance entre l'élément et une origine de faisceau.

10. Système d'imagerie par ultrasons, le système comprenant :
i. un réseau matriciel d'éléments transducteurs (200) ; et
ii. des circuits comprenant un circuit intégré spécifique à une application, ASIC, (100) intégré au réseau matriciel d'éléments transducteurs (200) et configuré pour :
a) amplifier, par l'ASIC, des signaux de réception de chaque élément de réseau de transducteurs ;
b) numériser, par l'ASIC, le signal de réception amplifié de chaque élément de réseau de transducteurs ;
c) appliquer, par l'ASIC, un retard et une pondération aux signaux de réception amplifiés et numérisés ; et
d) additionner, par l'ASIC, sur l'ensemble des éléments de transducteurs du réseau matriciel, les signaux de réception amplifiés, numérisés, retardés et pondérés afin de former un faisceau de réception focalisé de manière dynamique.
